**Europäisches Patentamt**

**European Patent Office**

**Office européen des brevets**

(11) Veröffentlichungsnummer: **0 407 841 A2**

# (12) EUROPÄISCHE PATENTANMELDUNG

(21) Anmeldenummer: **90112521.1**

(22) Anmeldetag: **30.06.90**

(51) Int. Cl.5: **C07C 41/06, C07C 43/10**

(30) Priorität: **14.07.89 DE 3923292**

(43) Veröffentlichungstag der Anmeldung:
**16.01.91 Patentblatt 91/03**

(84) Benannte Vertragsstaaten:
**BE DE FR GB IT NL**

(71) Anmelder: **EC ERDÖLCHEMIE GMBH**
**Postfach 75 20 02**
**D-5000 Köln 71(DE)**

(72) Erfinder: **Schleppinghoff, Bernhard, Dr.**
**Adolf-Kolping-Strasse 5**
**D-4047 Dormagen(DE)**
Erfinder: **Stüwe, Arnd, Dr.**
**Berta-von-Suttner-Strasse 34**
**D-5090 Leverkusen 1(DE)**
Erfinder: **Niederberger, Hans-Ludwig, Dr.**
**Goethestrasse 63**
**D-4047 Dormagen(DE)**
Erfinder: **Scheef, Hans-Volker**
**Goethestrasse 69**
**D-4047 Dormagen(DE)**
Erfinder: **Grub, Joachim, Dr.**
**Ostpreussenallee 10**
**D-4047 Dormagen(DE)**

(74) Vertreter: **Müller, Gerhard, Dr.**
**BAYER AG Konzernverwaltung RP**
**Patentabteilung**
**D-5090 Leverkusen 1 Bayerwerk(DE)**

(54) **Verfahren zur Herstellung von Äthern aus C4-C7-tert.-Alkenen und zwei- bis sechswertigen Alkoholen.**

(57) Ether aus C4-C7-tert.-Alkenen und zwei- bis sechswertigen Alkoholen werden an sauren Veretherungskatalysatoren und in Gegenwart von 20 bis 150 Gew.-%, bezogen auf das einzusetzende Gemisch aus tert.-Alken und mehrwertigem Alkohol, eines oder mehrerer der zu bildenden Ether hergestellt.

EP 0 407 841 A2

# VERFAHREN ZUR HERSTELLUNG VON ETHERN AUS C₄-C₇-TERT.-ALKENEN UND ZWEI BIS SECHSWERTI-GEN ALKOHOLEN

Die vorliegende Erfindung betrifft die Herstellung von tert.-Alkylethern von höheren als einwertigen Alkoholen

Es ist bekannt, tert.-Alkene beispielsweise an sauren Kationenaustauschern mit niederen Alkoholen zu verethern. Die auf diese Weise zugänglichen Methyl-tert.-butyl-ether (MTBE) und tert.-Amyl-methyl-ether (TAME) haben als Zusätze zu klopffestem Benzin Bedeutung erlangt. Das tert.-Alken wird hierbei vielfach nicht als reine Substanz eingesetzt, sondern im Gemisch mit anderen gesättigten und ungesättigten Kohlenwasserstoffen, wie sie als verschiedene Destillatschnitte bzw. Extraktionsraffinate in petrochemischen Anlagen und Raffinerien vorliegen. Aus solchen Gemischen heraus wird das tert.-Alken selektiv umgesetzt.

Es wurde nun ein Verfahren zur Herstellung von Ethern aus C₄-C₇-tert.-Alkenen und zwei- bis sechswertigen Alkoholen an sauren Veretherungskatalysatoren gefunden, das dadurch gekennzeichnet ist, daß man die Veretherung in Gegenwart von 20 bis 150 Gew.-%, bezogen auf das einzusetzende Gemisch aus tert.-Alken und mehrwertigem Alkohol, eines oder mehrerer der zu bildenden Ether durchführt.

C₄-C₇-tert.-Alkene für das erfindungsgemäße Verfahren sind i-Buten, 2-Methyl-buten-1, 2-Methyl-buten-2, die isomeren Hexene und Heptene, sofern sie ein tertiäres C-Atom an der Doppelbindung tragen. Auch solche isomeren C₅-C₇-Alkene sind einsetzbar, bei denen die Doppelbindung nicht am tertiären C-Atom sitzt, sofern unter den Reaktionsbedingungen eine Umlagerung zu tert.-Olefinen mit am tertiären C-Atom ständiger Doppelbindung stattfindet. Solche Alkene können auch als Gemisch mehrerer von ihnen eingesetzt werden. Solche tert.-Alkene werden im allgemeinen als Gemisch eines oder mehrerer von ihnen mit gesättigten und anderen ungesättigten Kohlenwasserstoffen eingesetzt, aus denen heraus sie selektiv umgesetzt werden. Beispiel für ein solches Kohlenwasserstoffgemisch mit einem Gehalt an i-Buten ist das sogenannte Raffinat I im Anschluß an die Butadien-Extraktion. Ein Beispiel für ein Kohlenwasserstoffgemisch mit einem Gehalt an tertiären 2-Methyl-butenen ist der sogenannte B(Benzol)-Vorlauf. Weitere Gemische solcher Art sind dem Fachmann bekannt. In bevorzugter Weise werden C₄- und/oder C₅-tert.-Alkene für die Veretherung eingesetzt.

Zwei- bis sechswertige Alkohole für das erfindungsgemäße Verfahren sind beispielsweise Diole, wie Ethylenglykol, Propandiol-1,2, Propandiol-1,3, Butandiol-1,2, Butandiol-1,3, Butandiol-1,4, Butandiol-2,3 und höhere Diole; Triole, wie Glyzerin, Trimethylolpropan und weitere Triole; Tetrole, Pentole und Hexole, wie Pentaerythrit und Zuckeralkohole mit vier bis sechs OH-Gruppen, wie sie aus der Reduktion von Zuckern entstehen; ferner Gemische aus solchen mehrwertigen Alkoholen, beispielsweise auch sogenannter Formit aus der Selbstkondensation von Formaldehyd, gefolgt von anschließender Reduktion (DE-OS 27 56 270, DE-OS 28 31 719, DE-OS 28 31 656); fernerhin Ether solcher mehrwertiger Alkohole, vorwiegend der Diole, untereinander, wie Diethylenglykol, Triethylenglykol, dimeres oder trimeres Propandiol, an denen auch nach der Veretherung endständig mindestens zwei OH-Gruppen vorhanden sind. In bevorzugter Weise werden zwei- und/oder dreiwertige Alkohole oder deren Ether untereinander eingesetzt. In besonders bevorzugter Weise wird Propandiol-1,2 eingesetzt.

Im erfindungsgemäßen Verfahren werden 0,1 bis 4 Mol tert.-Alken pro Äquivalent OH-Gruppe eingesetzt. In bevorzugter Weise werden 0,25 bis 2, in besonders bevorzugter Weise 0,5 bis 1,3 Mol tert.-Alken pro Äquivalent OH-Gruppe eingesetzt.

Die Veretherung wird bei 20 bis 90°C, bevorzugt bei 40 bis 80°C, besonders bevorzugt bei 45 bis 70°C, unter einem solchen Druck durchgeführt, daß das Veretherungs gemisch auch bei Verwendung niedrig siedender Alkene oder solche Alkene enthaltender Kohlenwasserstoffgemische in der flüssigen Phase verbleibt. Ein solcher Druck beträgt im allgemeinen 1 bis 8 bar, bevorzugt 1 bis 5 bar, und kann durch aufgedrückten Stickstoff oder in besonders bevorzugter Weise als der sich automatisch einstellende Eigendruck des Systems angewandt werden.

Die Veretherung wird an sauren Veretherungskatalysatoren durchgeführt. Solche sauren Veretherungskatalysatoren sind dem Fachmann bekannt. Hierzu zählen sowohl lösliche Veretherungskatalysatoren, wie Schwefelsäure, Phosphorsäure oder saure Metallsalze sowie starke organische Säuren, wie Toluolsulfonsäure oder Halogenessigsäuren, in bevorzugter Weise jedoch die unlöslichen sauren Veretherungskatalysatoren. Solche unlöslichen Veretherungskatalysatoren können mineralische Katalysatoren oder Kationenaustauscher in der H+-Form sein. Mineralische Veretherungskatalysatoren sind beispielsweise saure Zeolithe, sauer eingestelltes $Al_2O_3$ oder $SiO_2$. In besonders bevorzugter Weise werden die Kationenaustauscher in der H+-Form eingesetzt, die aus ethylenisch ungesättigten Monomeren, wie Styrol oder Acrylsäurederivaten und Vernetzern, wie Divinylbenzol, aufgebaut sind. Ferner kann es sich hierbei um Phenolformaldehydharze handeln. Die sauren, Kationen austauschenden Gruppen sind hierbei phenolische OH-Gruppen, Carboxylgruppen (beispielsweise die der Acrylsäurederivate, gegebenenfalls nach Verseifung) und Sulfonsäuregruppen, die durch nachträgliche Sulfonierung der im vernetzten Polymer vorhandenen aromatischen Kerne entstehen.

In besonders bevorzugter Form werden Sulfonsäuregruppen enthaltende Styrol-Divinylbenzol-Harze in der H+-Form eingesetzt. Solche Katalysatoren und ihre Herstellung sind dem Fachmann bekannt.

Diese Veretherungskatalysatoren werden mit einer LHSV (Liquid Hourly Space Velocity) beaufschlagt, die Werte von 0,1 bis 10 l umzusetzendes Ausgangsgemisch pro Liter Katalysator im trockenen Zustand pro Stunde, bevorzugt 0,5 - 5 l/l•h, beträgt.

Im erfindungsgemäßen Verfahren können je nach dem eingesetzten Verhältnis von tert.-Alken zu OH-Gruppen Ether der genannten mehrwertigen Alkanole erhalten werden, in denen eine oder mehrere der vorhandenen OH-Gruppen verethert sind. Am Beispiel von eingesetztem Propandiol-1,2 und i-Buten (beispielsweise aus Raffinat I) kann man sowohl 1-tert.-Butoxy-2-propanol, 2-tert.-Butoxy-1-propanol als auch 1,2-Di-tert.-butoxy-propan erhalten.

Es ist ein Kennzeichen des erfindungsgemäßen Verfahrens, daß dem Einsatzgemisch aus $C_4$-$C_7$-tert.-Alkenen und zwei-bis sechswertigen Alkoholen solche Ether in einer Menge von 20 bis 150 Gew.-%, bevorzugt von 40 bis 100 Gew.-%, solcher Ether, bezogen auf das gesamte einzusetzende Gemisch aus Alkenen und mehrwertigen Alkoholen zugesetzt werden. In bevorzugter Weise werden solche Ether dem einzusetzenden mehrwertigen Alkohol zugesetzt; danach wird das so erhaltene Gemisch mit den tert.-Alkenen oder den solche tert.-Alkene enthaltenden Kohlenwasserstoffgemischen vereinigt und dem Veretherungsreaktor zuge führt. Falls die tert.-Alkene in reiner oder hochkonzentrierter Form eingesetzt werden, können Gewichtsprozente an solchen Ethern im unteren Teil des angegebenen Bereichs gewählt werden; falls Gemische der tert.-Alkene mit anderen Kohlenwasserstoffen eingesetzt werden, wählt man Ethermengen im oberen Teil des angegebenen Bereichs. Ein solches umzusetzendes Gesamtgemisch ist erfindungsgemäß dadurch ausgezeichnet, daß es bereits vor dem Eintritt in den Veretherungsreaktor eine einzige flüssige Phase darstellt.

Der Veretherungsstufe wird, falls erforderlich, eine Neutralisationsstufe nachgeschaltet, in der durch basische anorganische Verbindungen oder Anionenaustauscher etwa vom Veretherungkatalysator mitgeschwemmte saure Verbindungen abgetrennt werden. Des weiteren kann in einer dem Fachmann bekannten Weise ein Teil des den Veretherungskatalysator verlassenden Gemisches erneut auf den Eingang zur Veretherungsstufe gegeben werden, um die exotherme Reaktionswärme besser beherrschen zu können. Des weiteren werden dem Veretherungsreaktor und gegebenenfalls der Neutralisationsstufe verschiedene Destillationskolonnen, teilweise als Vakuumdestillationskolonnen eingerichtet, nachgeschaltet, um das Veretherungsgemisch in die gewünschten Bestandteile aufzutrennen.

Für den Fall, daß in dem tert.-Alkene enthaltenden Kohlenwasserstoffgemisch neben Olefinen auch Diolefine vorliegen, kann es wünschenswert sein, diese Diolefine selektiv zu Olefinen zu hydrieren. Dies kann vor Eintritt in die Veretherung erfolgen oder simultan zur Verethe rung; im letzteren Fall (wird beispielsweise ein saurer Kationenaustauscher als Katalysator eingesetzt, der neben den sauren $SO_3H$-Gruppen eine Dotierung mit elementarem Edelmetall (z.B. Pd) aufweist. Zusätzlich muß hierbei das Veretherungsgemisch am Katalysator mit $H_2$ beaufschlagt werden (EP 87 658).

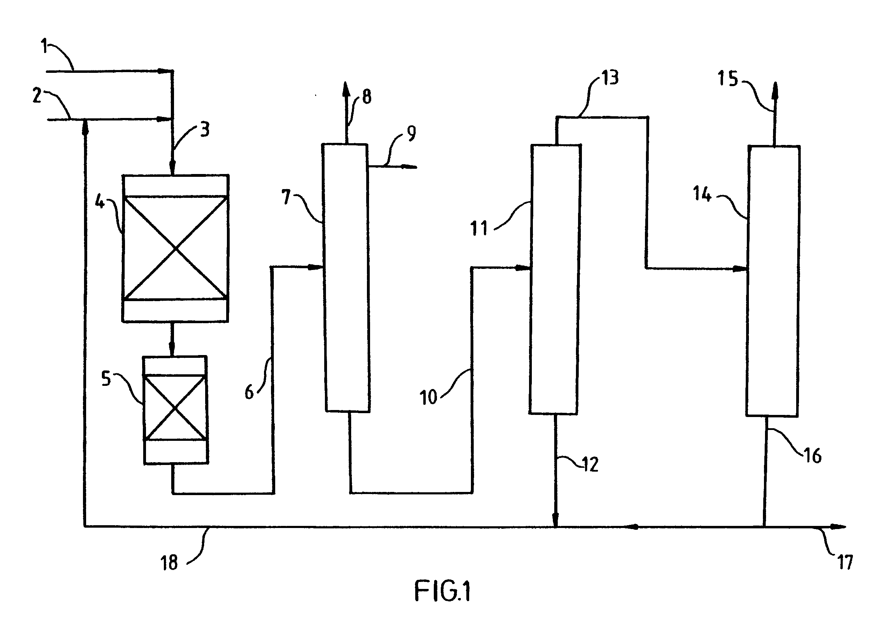

Anhand von Fig. 1 sei das erfindungsgemäße Verfahren am Beispiel der Umsetzung von Raffinat I mit einem Gehalt an i-Buten mit Propandiol-1,2 dargestellt: Raffinat I (1) und Propandiol-1,2 (2) fließen gemeinsam über die Leitung (3) in den Veretherungsreaktor (4). Dem Propandiol-1,2 war vorher über die Leitung (18) ein Teil des erhaltenen Ethergemisches sowie etwa nicht vollständig umgesetztes Propandiol-1,2 zugesetzt worden. Alternativ könnte dieser Zusatz jedoch auch aus einem Vorratstank, beispielsweise von einem vorangegangenen Produktionslauf, genommen werden; es ist jedoch bevorzugt, ohne solche Vorratshaltung mit simultanem Kreislauf zu fahren, bis sich ein stationärer Zustand eingestellt hat, und die produzierten Ether neben der Recyclisierung als Produkt zu entnehmen. (4) Nachgeschaltet befindet sich eine Neutralisationsstufe (5), von der das Veretherungsgemisch über die Leitung (6) einem Stripper (7) zuläuft, in welchem über Kopf das von i-Buten ganz oder teilweise abgereicherte Kohlenwasserstoffgemisch abströmt (8). Im Falle einer weitgehenden (bis vollständigen) Entnahme des i-Butens handelt es sich bei diesem Kopfstrom um das sogenannte Raffinat II. (7) enthält weiterhin unterhalb des Kolonnenkopfes bei (9) die Entnahmemöglichkeit für einen Seitenstrom, an welchem tert.-Butanol und Wasser entnommen werden können, wenn durch die Ausgangsprodukte etwas Wasser in das Veretherungsgemisch eingebracht worden war. Bei (9) können weiterhin geringe Mengen Oligomere von i-Buten entnommen werden. Die schwer flüchtigen Bestandteile des Veretherungsgemisches verlassen (7) über die Leitung (10) und werden einer ersten Vakuumdestillationskolonne (11) zugeführt, über deren Kopf durch die Leitung (13) das Gemisch der Mono- und Diether entnommen wird, während am Boden über die Leitung (12) etwa nicht umgesetztes Propandiol-1,2 entnommen wird. Das Gemisch der Ether wird über die Leitung (13) einer zweiten Vakuumdestillationskolonne (14) zugeführt, in der es in das leichter flüchtige 1-tert.-Butoxy-2-propanol (15) und das Gemisch des schwerer flüchtigen 2-tert.-Butoxy-1-propanol und 1,2-Di-tert.-butoxy-propan aufgetrennt wird. Dieses zuletzt genannte Gemisch verläßt (14) über die Leitung (16) und wird zum Teil bei (17) als Produkt entnommen und zum Teil gemeinsam mit dem über Leitung (12) zuströmenden Sumpfprodukt von (11) über die Leitung (18) dem frisch einzusetzenden Propandiol-1,2 zugemischt. Selbstverständlich ist es auch möglich, auf die destillative Trennung in der zweiten Vakuumdestillationskolonne (14) zu verzichten, und ein Gemisch aller drei der genannten Ether über die Leitung (18) zurückzuführen. Dies ist insbesondere dann angezeigt, wenn als zu entnehmendes Produkt gleichzeitig das Gemisch aller drei der genannten Ether gewünscht wird.

Die erfindungsgemäße Herstellung von Ethern aus C$_4$-C$_7$-tert.-Alkenen und zwei- bis sechswertigen Alkoholen kann verschiedenen interessanten technischen Zwecken dienen. So können die erhältlichen Ether einzeln oder als Gemisch als interessante Lösungsmittel, Detergentien und bei noch vorhandenen freien OH-Gruppen als Lackrohstoffe verwendung finden. Eine Auftrennung in Einzelkomponenten ist nicht immer erforderlich, kann aber, wie oben gezeigt wurde, durchgeführt werden.

Eine weitere wichtige technische Anwendung besteht im Einsatz der erfindungsgemäß erhältlichen Ether zur Spaltung und Rückgewinnung von reinen tert.-Alkenen. Bei diesem Verwendungszweck ist es von außerordentlichem Vorteil, daß die zurückbleibenden alkoholischen Spaltprodukte aufgrund ihrer Siedepunktslage definitiv im Sumpf einer der Spaltung nachgeschalteten Trennkolonne verbleiben. Des weiteren tritt keine unerwünschte Bildung von Ethern einwertiger Alkanole auf, die beim Einsatz von MTBE oder TAME als zu spaltende Ether stets zu befürchten sind. Insbesondere bei den genannten Methylethern ist die Bildung des Dimethylethers (DME) besonders gefürchtet, weil sie zum einen an den Spaltkatalysatoren recht einfach verläuft und daher in nennenswerter Menge auftritt und weil DME wegen seiner Siedepunktslage schwer aus dem zu gewinnenden reinen tert.-Alken entfernt werden kann.

Eine noch weitere wichtige industrielle Anwendung des erfindungsgemäßen Veretherungsverfahrens ist die vollständige Abtrennung von tert.-Alkenen aus den sie enthaltenden Kohlenwasserstoffgemischen. Dies ist besonders wichtig, weil beispielsweise zur Weiterverwendung von Raffinat II oder C$_5$-C$_7$-Raffinaten, die hierzu äquivalent sind, eine besonders weitgehende Befreiung von den ursprünglich darin enthaltenen tert.-Alkenen gefordert wird. Hier ist es nun erfindungsgemäß möglich, einen hohen Überschuß an OH-Gruppen einzusetzen, um eine solche möglichst weitgehende Abtrennung der tert.-Alkene aus den sie enthaltenden Kohlenwasserstoffströmen zu ermöglichen, da bei der Abtrennung der übrig bleibenden Kohlenwasserstoffe, beispielsweise in (7) von Fig. 1 die entstandenen Ether und die überschüssig eingesetzten höherwertigen Alkanole definitiv im Sumpf verbleiben. In diesem Falle wird das molare Verhältnis von tert.-Alken pro Äquivalent OH-Gruppe im unteren Teil des obengenannten allgemeinen Bereichs bleiben, beispielsweise bei 0,1 bis 1 Mol, bevorzugt 0,1 bis 0,8 Mol, tert.-Alken pro Äquivalent OH-Gruppe. Selbstverständlich kann neben dem zu recyclisierenden mehrwertigen Alkohol und dem ebenfalls erfindungsgemäß zu recyclisierenden Anteil Ether auch überschüssiger gebildeter Ether als Produkt entnommen und gegebenenfalls destillativ in seine Einzelkomponenten aufgetrennt werden.

Beispiel 1

Die Veretherung der Glykole mit 1-Buten beziehungsweise i-Amylen wurde an einem starksauren, makroporösen Kationenaustauscher (Styrol-Divinylbenzol-Harz mit SO$_3$H-Gruppen, wie z.B. SPC-118 von Bayer AG) durchgeführt.

Die Veretherung wurde vergleichend zu diesem Katalysator in Gegenwart von H$_2$ an einem Pd-dotierten, starksauren makroporösen Kationenaustauschern der obigen Art durchgeführt, wenn im Einsatzprodukt der zu verethernden Ströme Diene (Butadien) in der Größenordnung bis 1 % vorlagen.

Für die Veretherung ist diese Variante nicht relevant. Sie läuft im gleichen Maße ab.

Die Veretherung des Monoethylenglykols (MEG) mit i-Buten-haltigem Raffinat wurde in einem Rohrreaktor in der Flüssigphase durchgeführt. Der Rohrreaktor hatte einen lichten Durchmesser von 20 mm und war mantelbeheizt. Er war mit einer Druckregelung ausgerüstet, das Reaktionsprodukt wurde in einem gekühlten Abscheider aufgefangen, das Abgas wurde über eine Gasuhr gemessen.

Der Reaktor war mit einer Kreislaufführung ausgerüstet. Die Mengenströme waren pumpengeregelt. Die Anlage wurde kontinuierlich betrieben, nach 48 Stunden kontinuierlicher Fahrweise wurden Analysen gezogen, diese wurden gaschromatographisch bestimmt.

Die Reaktionsführung war folgendermaßen:

C$_4$-Einsatz : flüssig

Glykol-Einsatz : flüssig

Produktflußrichtung : von unten nach oben
Reaktor : mit Kreislauf (Frischeinsatz:Kreislauf = 1 : 1)
Abscheider : gekühlt mit Standregelung
Abgas : Kondensation der Gasphase
Folgende Reaktionsbedingungen wurden eingestellt:
Reaktionsdruck : 15 bar
LHSV / Temperatur : 2/65° C
Frischeinsatz/Kreislauf : 1 : 1
Die erhaltenen Ergebnisse sind in der Tabelle 1 aufgelistet:

| Komponente | Einsatz | Reaktionsprodukt |
|---|---|---|
| Butane [g/h] | 65.0 | 65.0 |
| i-Buten [g/h] | 226.0 | 27.2 |
| n-Butene [g/h] | 208.0 | 208.0 |
| MEG [g/h] | 251.0 | 70.5 |
| MEG-ME [g/h] | - | 267.9 |
| MEG-DE [g/h] | - | 111.4 |

Der i-Buten-Umsatz betrug : 88 %,
der MEG-Umsatz betrug : 72 %,
die Selektivität zum Monoether : 78 % und
die Selektivität zum Diether : 22 %.
MEG - Monoethylenglykol
MEG-ME = Monoethylenglykol-mono-tert.-butylether
MEG-DE = Monoethylenglykol-di-tert.-butylether
Bei einem Verhältnis von Einsatz zu kreislaufendem Ausgangsmaterial = 1:1 lagen 51,4 Gew.-% Ether, bezogen auf eingesetztes i-Buten + MEG, vor.

Beispiel 2

Der Versuch wurde analog zu Beispiel 1 gefahren, jedoch wurde das Molverhältnis Monoethylenglykol zu 1-Buten geändert, um einen höheren Anteil an Diether zu erzielen.
Die Ergebnisse sind in der nachfolgenden Tabelle aufgeführt.

| Komponente | Einsatz | Reaktionsprodukt |
|---|---|---|
| Butane [g/h] | 65.0 | 65.0 |
| i-Buten [g/h] | 226.0 | 70.0 |
| n-Butene [g/h] | 208.0 | 208.0 |
| MEG [g/h] | 100.0 | 4.0 |
| MEG-ME [g/h] | - | 36.5 |
| MEG-DE [g/h] | - | 215.5 |

Der i-Buten-Umsatz betrug : 69.0 %,
der MEG-Umsatz betrug : 96.0 %,
die Selektivität zum Monoether : 20.0 % und
die Selektivität zum Diether : 80.0 %.
Bei einem Verhältnis von Einsatz:Kreislauf = 1:1 lagen 63 Gew.-% Ether, bezogen auf die Summe i-Buten + MEG, vor.

Beispiel 3

EP 0 407 841 A2

Der Versuch wurde analog zu Beispiel 1 durchgeführt, anstelle von Monoethylenglykol wurde Diethylenglykol eingesetzt.

Die Ergebnisse sind in der nachfolgenden Tabelle aufgeführt.

| Komponente | Einsatz | Reaktionsprodukt |
|---|---|---|
| Butane [g/h] | 65.0 | 65.0 |
| i-Buten [g/h] | 226.0 | 22.5 |
| n-Butene [g/h] | 208.0 | 208.0 |
| DEG [g/h] | 428.0 | 107.0 |
| DEG-ME [g/h] | - | 392.3 |
| DEG-DE [g/h] | - | 132.3 |

DEG = Diethylenglykol

DEG-ME = Diethylenglykol-mono-tert.-butylether

DEG-DE = Diethylenglykol-di-tert.-butylether

Der i-Buten-Umsatz betrug : 90.0 %,

der DEG-Umsatz betrug : 75.0 %,

die Selektivität zum Monoether : 80.0 % und

die Selektivität zum Diether : 20.0 %.

Bei einem Verhältnis von Einsatz zu Kreislauf = 1:1 lagen 67 Gew.-% Ether, bezogen auf die Summe i-Buten + DEG, vor.

Beispiel 4

Der Versuch wurde analog zu Beispiel 1 durchgeführt, anstelle von Monoethylenglykol wurde jedoch Monopropylenglykol eingesetzt.

Die Ergebnisse sind in der nachfolgenden Tabelle aufgeführt:

| Komponente | Einsatz | Reaktionsprodukt |
|---|---|---|
| Butane [g/h] | 65.0 | 65.0 |
| i-Buten [g/h] | 226.0 | 19.7 |
| n-Butene [g/h] | 208.0 | 208.0 |
| MPG [g/h] | 307.0 | 73.3 |
| MPG-ME [g/h] | - | 324.2 |
| MPG-DE [g/h] | - | 115.4 |

Der i-Buten-Umsatz betrug : 91.3 %,

der MPG-Umsatz betrug : 76.1 %,

die Selektivität zum Monoether : 80.0 % und

die Selektivität zum Diether : 20.0 %

MPG = Monopropylenglykol

MPG-ME = Monopropylenglykol-mono-tert.-butylether

MPG-DE = Monopropylenglykol-di-tert.-butylether

Bei einem Verhältnis von Einsatz zu Kreislauf = 1:1 lagen 70 Gew.-% Ether, bezogen auf die Summe i-Buten + MPG, vor.

Beispiel 5

Der Versuch wurde analog zu Beispiel 4 durchgeführt, anstelle von 1-Buten wurde i-Amylen eingesetzt.

Die Ergebnisse sind in der nachfolgenden Tabelle aufgeführt:

6

| Komponente | Einsatz | Reaktionsprodukt |
|---|---|---|
| Rest-KW [g/h] | 500.0 | 500.0 |
| i-Amylen [g/h] | 85.0 | 7.5 |
| MPG [g/h] | 90.0 | 20.0 |
| MPG-MAE [g/h] | - | 107.7 |
| MPG-DAE [g/h] | - | 39.8 |

Der i-Amylen-Umsatz betrug : 90.6 %,
der MPG-Umsatz betrug : 77.8 %,
die Selektivität zum Monoether : 80.0 % und
die Selektivität zum Diether : 20.0 %
MPG = Monopropylenglykol
MPG-MAE = Monopropylenglykol-mono-tert.-amylether
MPG-DAE = Monopropylenglykol-di-tert.-amylether.

Bei einem Verhältnis von Einsatz zu Kreislauf = 1:1 lagen 72,8 Gew.-% Ether, bezogen auf die Summe i-Amylen + MPG, vor.

**Ansprüche**

1. Verfahren zur Herstellung von Ethern aus $C_4$-$C_7$-tert.-Alkenen und zwei- bis sechswertigen Alkoholen an sauren Veretherungskatalysatoren, dadurch gekennzeichnet, daß man die Veretherung in Gegenwart von 20 bis 150 Gew.-%, bezogen auf das einzusetzende Gemisch aus tert.-Alken und mehrwertigem Alkanol, eines oder mehrerer der zu bildenden Ether durchführt.

2. Verfahren nach Anspruch 1, dadurch gekennzeichnet, daß in Gegenwart von 40 bis 100 Gew.-% des zu bildenden Ethers gearbeitet wird.

3. Verfahren nach Anspruch 1, dadurch gekennzeichnet, daß $C_4$- und/oder $C_5$-Alkene eingesetzt werden.

4. Verfahren nach Anspruch 1, dadurch gekennzeichnet, daß zwei- und/oder dreiwertige Alkohole oder deren Ether untereinander eingesetzt werden.

5. Verfahren nach Anspruch 1, dadurch gekennzeichnet, daß 0,1 bis 4 Mol tert.-Alken pro Äquivalent OH-Gruppe eingesetzt werden.

6. Verfahren nach Anspruch 5, dadurch gekennzeichnet, daß 0,25 bis 2, bevorzugt 0,5 bis 1,3 Mol tert.-Alken pro Äquivalent OH-Gruppe eingesetzt werden.

7. Verfahren nach Anspruch 1, dadurch gekennzeichnet, daß bei 20 bis 90° C, bevorzugt 40 bis 80° C, besonders bevorzugt 45 bis 70° C, gearbeitet wird.

8. Verfahren zur Herstellung von Ethern nach Anspruch 1 und zur gleichzeitigen Abtrennung von $C_4$-$C_7$-tert.-Alkenen aus diese enthaltenden Kohlenwasserstoffströme, dadurch gekennzeichnet, daß 0,1 bis 1 Mol, bevorzugt 0,1 bis 0,8 Mol, tert.-Alken pro Äquivalent OH-Gruppe eingesetzt werden und daß das Veretherungsgemisch in einer nachgeschalteten Destillation in ein an tert.-Alken freies Kopfprodukt und in ein teilweise verethertes Sumpfprodukt getrennt wird.

9. Verfahren zur Herstellung von 1-tert.-$C_4$-$C_5$-Alkoxy-2-propanol nach Anspruch 1, dadurch gekennzeichnet, daß man ein Veretherungsgemisch aus Propandiol-1,2 und $C_4$-$C_5$-tert.-Alken destillativ in 1-tert.-$C_4$-$C_5$-Alkoxy-2-propanol als Kopfprodukt und ein Gemisch aus 2-tert.-$C_4$-$C_5$-Alkoxy-1-propanol, 1,2-Di-tert.-$C_4$-$C_5$-alkoxy-propan und Propandiol-1,2 als Sumpfprodukt trennt und das Sumpfprodukt in die Veretherung zurückführt.

FIG.1